# EUROPEAN PATENT SPECIFICATION

(11) **EP 1 948 817 B1**
(45) Date of publication and mention of the grant of the patent: **24.02.2010**
(21) Application number: 06777059.4
(22) Date of filing: 24.08.2006
(51) Int. Cl.: C12P 21/02, C07K 1/04

(54) **CELL-FREE IN VITRO TRANSCRIPTION AND TRANSLATION OF MEMBRANE PROTEINS INTO TETHERED PLANAR LIPID LAYERS**
ZELLFREIE IN-VITRO-TRANSKRIPTION UND TRANSLATION VON MEMBRANPROTEINEN IN GEBUNDENE PLANARE LIPIDSCHICHTEN
TRANSCRIPTION ET TRADUCTION IN VITRO ACELLULAIRES DE PROTEINES MEMBRANAIRES EN COUCHES LIPIDIQUES PLANAIRES ATTACHEES

(30) Priority: 28.10.2005 EP 05023705
(43) Date of publication of application: 30.07.2008
(73) Proprietor: Max-Planck-Gesellschaft zur Förderung der Wissenschaften e.V., 80539 München (DE); SINNER, Eva-Kathrin, 64289 Darmstadt (DE)
(72) Inventor: SINNER, Eva-Kathrin, 64289 Darmstadt (DE); WILTSCHI, Birgit, 85521 Ottobrunn (DE); LEMKER, Eva, 70794 Filderstadt (DE); ROBELEK, Rudolf, 93055 Regensburg (DE)
(74) Representative: Dey, Michael
(86) International application number: PCT/EP2006/008318
(87) International publication number: WO 2007/048459

(56) References cited:
- NOIREAUX VINCENT ET AL: "A vesicle bioreactor as a step toward an artificial cell assembly." PROCEEDINGS OF THE NATIONAL ACADEMY OF SCIENCES OF THE UNITED STATES OF AMERICA. 21 DEC 2004, vol. 101, no. 51, 21 December 2004 (2004-12-21), pages 17669-17674, XP002412221 ISSN: 0027-8424
- NOIREAUX VINCENT ET AL: "Toward an artificial cell based on gene expression in vesicles." PHYSICAL BIOLOGY., vol. 2, no. 3, 15 September 2005 (2005-09-15), pages P1-P8, XP002412220 ISSN: 1478-3967
- DATABASE WPI Week 200549 Derwent Publications Ltd., London, GB; AN 2005-482167 XP002412235 & JP 2005 168316 A (TOYOBO KK) 30 June 2005 (2005-06-30)
- SINNER E-K ET AL: "Incorporation of integrins into artificial planar lipid membranes: characterization by plasmon-enhanced fluorescence spectroscopy" ANALYTICAL BIOCHEMISTRY, ACADEMIC PRESS, NEW YORK, NY, US, vol. 333, no. 2, 15 October 2004 (2004-10-15), pages 216-224, XP004573008 ISSN: 0003-2697 cited in the application
- NAUMANN R ET AL: "THE PEPTIDE-TETHERED LIPID MEMBRANE AS A BIOMIMETIC SYSTEM TO INCORPORATE CYTOCHROME C OXIDASE IN A FUNCTIONALLY ACTIVE FORM" BIOSENSORS & BIOELECTRONICS, ELSEVIER SCIENCE PUBLISHERS, BARKING, GB, vol. 14, no. 7, 1999, pages 651-662, XP001038493 ISSN: 0956-5663 cited in the application

## Description

The present invention relates to membranes, in particular, to synthetic membranes, having membrane proteins incorporated therein and, in particular, to the cell-free in vitro transcription and translation of membrane proteins into membranes, e.g. into tethered planar lipid layers. In particular, the present invention provides a new method for the preparation of such membranes. The membranes, e.g., can be used for investigation of membrane receptor/ligand interactions.

A detailed investigation of membrane receptor/ligand interaction is a prerequisite for understanding complex biological pathways involving cell membranes. To perform such investigations, efficient and reproducible in vitro assay systems are required to characterize specific receptor/ligand binding interaction in isolation from other interactions, in which the receptor may be engaged in natural environments.

Therefore, model systems for biological membranes have been developed such as liposomes, planar black lipid membranes (BLMs) as well as solid-supported membranes such as solid-supported lipid bilayers and tethered lipid bilayers. Tethered lipid membranes (tBLMs) are solid-supported lipid films with hydrophilic spacer groups such as peptide, polyethylene glycol or sugar groups, tethered covalently to a support. To incorporate membrane protein into such model membrane systems, isolation of membrane proteins and reconstitution into the membrane system has been necessary so far. Thus, for example, forming of a phospholipid monolayer on a solid support and subsequently subjecting this monolayer to lipid vesicles containing acetylcholine receptor (AChR) leads to the incorporation of the acetylcholine receptor into a bilipid membrane, wherein the second layer is formed from lipids contained in the lipid vesicle (E. K. Schmidt et al., Biosensors and Bioelectronics 13 (1998) 585-591). R. Naumann et al. (Biosensors and Bioelectronics 14 (1999) 651-662 describe the incorporation of cytochrome c oxidase in functionally active form into a peptide-tethered lipid membrane. Liposomes comprising phosphatidylcholine are spread on a thiopeptide-lipid monolayer to form a peptide-tethered lipid bilayer membrane. This membrane is then incubated with isolated cytochrome c oxidase.

A similar approach was reported for incorporation of integrins into artificial planar lipid membranes (E. K. Sinner, Analytical Biochemistry 333 (2004) 216-224). In this approach, integrins were incorporated into a lipid-functionalized peptide layer by vesicle spreading. Also with this approach membrane protein-containing vesicles had to be prepared first, requiring preparation and isolation of the membrane protein.

A problem in the case of the hitherto used manufacturing methods was that the membrane proteins had to be isolated first. The native activity of the proteins was often lost thereby. Further, incorporation into the membranes, e.g. in the case of vesicle spreading, was often effected randomly, however, not directed. This led to non-optimal test systems, in which the interaction and orientation of the proteins with and in the membrane as well as its effect on interactions with ligands cannot be investigated.

Therefore, it was an object of the invention to provide an improved method for the preparation of membranes having membrane proteins incorporated therein, especially a method, wherein the membrane proteins do not have to be isolated first.

According to the invention this object is achieved by a process for the preparation of membrane proteins incorporated in a membrane, comprising the steps:
(i) providing a membrane, wherein the membrane is a surface bound membrane,
(ii) applying a cell-free expression system and a nucleic acid coding for the membrane proteins to the membrane, and
(iii) expression of the membrane proteins, which are integrated into the membrane.

The method of the invention allows to incorporate membrane proteins in native functional form into membranes, in particular, into synthetic membranes, without the membrane proteins having to be isolated first. Further, it was surprisingly found that when carrying out the method of the invention the proteins expressed with the cell-free expression system are incorporated into membranes in directed form. According to the invention, thus, functional active proteins can be obtained in membranes without requiring previous isolation. Further, the membranes produced according to the invention have high stability, since due to the use of cell-free expression systems, for example, no protein-degrading proteases are present in the system.

It is presumed that due to expression of the membrane proteins with a cell-free expression system in the presence of a membrane the latter acts as a quasi-substitute for the endoplasmatic reticulum. Cell-free expression systems have been used to express soluble proteins in aqueous systems, whereby in many cases no natively active proteins but denatured proteins, e.g. in the form of inclusion bodies, are obtained. Surprisingly, it has been found that by expression of proteins in such cell-free expression system in the presence of a membrane, preferably a synthetic membrane the proteins are incorporated into the membrane and, moreover, are present therein in functional form. Incorporation into synthetic membranes is even more surprising, since incorporation mechanisms for membrane proteins which possibly are still present in naturally occurring membranes or in membranes of natural origin cannot be present in synthetic membranes. For example, microsome preparations which contain membranes derived from natural sources can have incorporation mechanisms for membrane proteins. Surprisingly, it has now been found by the inventors, however, that functional incorporation of membrane proteins can also be accomplished excellently with synthetic membranes no longer having such mechanisms.

The membrane employed is a surface bound membrane. It is also preferred to use a planar membrane. Particularly preferred are tethered membranes.

According to a particularly preferred embodiment of the invention, first, a synthetic membrane is provided. The synthetic membrane preferably is a planar membrane and/or has a vesicle-composite architecture. Especially preferably, it is a tethered membrane applied onto a carrier.

Tethering of membranes can be effected, for example, via peptide anchors, PEG anchors, sugar anchors, silane anchors, silane/thiol anchors or polymer anchors, preferably via peptide anchors. Binding or tethering of the membrane to the surface is preferably accomplished using hydrophilic spacer molecules.

Suitable carrier surfaces, for example, are metal or metal oxide, especially gold or silica surfaces. In the case of gold surfaces, the membrane is attached to the gold surface preferably by Au-S interaction. The surfaces can be dielectric or non-dielectric. In one embodiment semi-conductor surfaces are used.

In a particularly preferred embodiment, peptides are used as hydrophilic spacer molecules for tethering lipid membranes. Suitable peptide spacer molecules preferably have a length of 3-100, preferably 4-30, in particular, 5-25 and even more preferably 15-20 amino acids. Further, the chosen sequences have a cysteine residue on one end. When using a gold surface, a monomolecular peptide layer can be obtained by self-assembly caused by strong gold-sulfur interaction of the preferred terminal N-cysteine moiety. The 19-mer peptide CSRARKQAASIKVAVSADR (P19) derived from the α-laminin subunit has proven particularly useful.

The membrane used according to the invention preferably is a synthetic membrane, i.e. not a membrane of natural origin. This is advantageous because actually model systems are obtained thereby, wherein specific desired properties can be analyzed and other potential interactions which might be caused by components of natural membranes are excluded. The membrane used according to the invention, therefore, preferably consists of synthetically produced lipids, in particular, phospholipids. However, it is also possible to employ natural membranes or fragments of natural membranes, e.g. microsomes. Especially preferably, the membrane comprises dimyristoylphosphatidylethanolamine (DMPE) and/or phosphatidylcholine.

The coupling of a first lipid layer to the tethering molecules such as peptides preferably is covalent coupling. If peptides are used as tethering molecules, coupling can be achieved, e.g. by covalently linking the peptides via their carboxy terminus to primary amino moieties of phospholipid molecules such as dimyristoylphosphatidylethanolamine. The coupling can be performed by any known coupling chemistry, e.g. by an active ester coupling procedure using EDC (N-ethyl-N'-(dimethylaminopropyl)carbodiimide) and NHS (N-hydroxysuccinimide). A second lipid layer is then formed by adding additional lipid molecules to the system, e.g. by vesicle fusion.

The membrane protein to be incorporated into a membrane according to the invention can be any membrane protein. Examples are G-protein coupled receptors such as odorant receptors, rhodopsin receptors, in particular bovine rhodopsin receptors, rhodopsin pheromone receptors, peptide hormone receptors, taste receptors, GABA receptors, opiate receptors, serotonin receptors, Ca²⁺ receptor, melanopsin, neurotransmitter receptors, ligand gated, voltage gated or mechanically gated such as acetylcholine (ACh) , nicotinic, adrener, norepinephrine, catecholamines, L-dopa, dopamine and serotonin - biogenic amines, endorphins/enkephalins - neuropeptide receptors, kinases such as serin/threonin kinases, cytoplasmic tyrosine kinases, receptor tyrosine kinases, phosphatases proteases, inactive kinases, porins/channels such as chloride channels, potassium channels, sodium channels, OMP proteins, ABC transporter (ATP-Binding Cassette - Transporter) such as amino acid transporter, Na-glucose transporter, Na⁺/iodide transporter , ion transporters such as Light Harvesting Complex, cytochrome c oxidase, ATP ase Na/K, H/K,Ca, cell adhesion receptors such as metallo proteases, integrins, catherins.

For subsequent examination and investigation of the system, the membrane protein preferably is coupled with a tag. The tag preferably is selected from epitopes which allow binding of a specific antibody thereto. Suitable tags, e.g. are VSV (vesicular stomatitis virus glycoprotein), a His tag, Strep tag, Flag tag, intein tag or GST tag. However, it is also possible to couple to the membrane protein a partner of a high affinity binding pair such as biotin or avidin or a label such as an enzyme label or a fluorescent label which allows direct determination of the membrane protein.

The tag then can be used to couple a detectable group such as a fluorescent group to the membrane protein. Examination of the membrane and, in particular, of the presence and activity of membrane proteins, for example, can be performed by Surface Plasmon Resonance Spectroscopy or Surface Plasmon-enhanced Fluorescence Spectroscopy (SPFS). These methods allow to monitor the assembling of lipid membranes and binding interactions of incorporated membrane proteins in real time. These methods also allow detection and monitoring of very few protein molecules within a membrane such as 10-10000, in particular, 100-1000 protein molecules.

An essential component of the present invention is the use of a cell-free expression system. By the cell-free expression system a nucleic acid coding for the desired membrane protein, optionally also coding for a tag, is transcribed and translated and, thus, the desired membrane protein is formed in situ and then immediately incorporated into the synthetic membrane. Such a cell-free expression system preferably is an in vitro transcription and translation system as described e.g. in US 5,324,637. Preferably, a eukaryotic cell-free extract is used as an expression system. Such expression systems are commercially available, e.g. as TNT® coupled transcription/translation system from Promega Corporation. However, it is also possible to use a prokaryotic cell-free expression system (e.g. RTS 100 E.coli hy kit™ from Roche Applied Science) or an archaic cell-free expression system (e.g. Sarma; E.M. Fleischmann, Cold Spring Harbour Press, ISBN 0-87969-438-6, Protocol 18, p. 133).

Preferably, the nucleic acid coding for the membrane protein and optionally for tags or other functional portions added to the membrane protein are added as cDNA to the cell-free expression system.

As described above, surprisingly, it was found that expression of the membrane proteins with a cell-free expression system, preferably a eukaryotic cell-free expression system, in the presence of a synthetic membrane leads to the integration and incorporation of the membrane proteins into the synthetic membrane, whereby the membrane proteins are in a functionally active form. Thus, the membranes obtainable according to the present invention are highly suitable as assay systems in research, in particular, for investigation of interactions between membrane proteins, such as receptors and their ligands. Therefore, al so disclosed is a synthetic membrane having incorporated therein a membrane protein, which synthetic membrane is obtainable by the process as described herein.

The weight ratio of membrane proteins incorporated into membrane lipids is preferably 1:1 to 1:10000, in particular, 1:100 to 1:1000.

Membranes having incorporated therein functionally active membrane proteins can be used as assay systems for determining the function and/or structure of membrane proteins and, in particular, for investigation of receptor/ligand interactions. However, they can also be used in sensor technology, e.g. as an odorant receptor. Further uses are warfare applications, detection of biotoxic, e.g. anthrax, toxic or explosive material, ion sensors, drugs sensors or amino acid sensors.

The invention is further explained by the accompanying Figures and the following Examples.

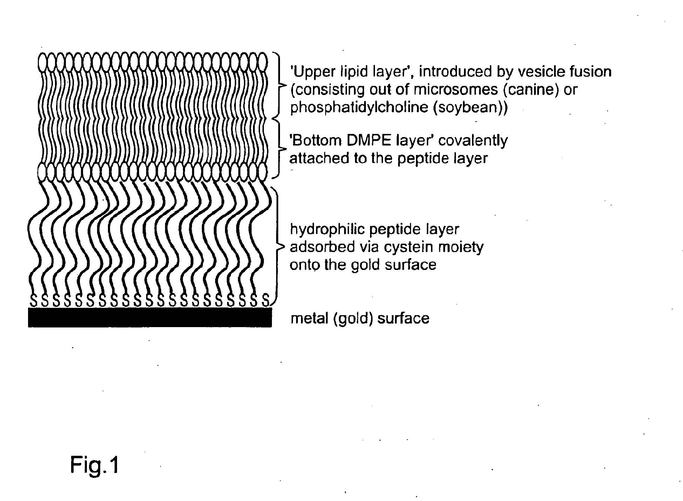

- Fig. 1: shows an experimental setup of a peptide layer tethered bilipid layer. The peptide layer is absorbed onto a gold surface of a support via the S atom of a cysteine moiety. To the peptide layer a DMPE layer is covalently attached. An upper lipid layer is introduced by vesicle fusion.
- Fig. 2: shows schematically the incorporation of GPCR molecules into a planar membrane system coupled to an anti-mouse-Cy5 antibody.

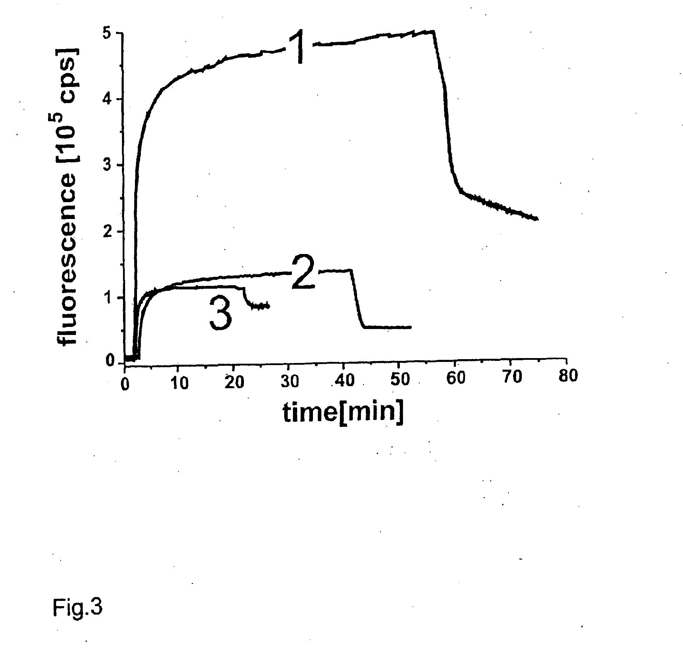

- Fig. 3: shows fluorescence signals for
1) addition of GPCR cDNA with N-terminal VSV tag,
2) addition of GPCR cDNA with C-terminal VSV tag, and
3) addition of TnT vector without insert.
The arrow indicates addition of Cy5 labeled secondary anti-VSV antibody.

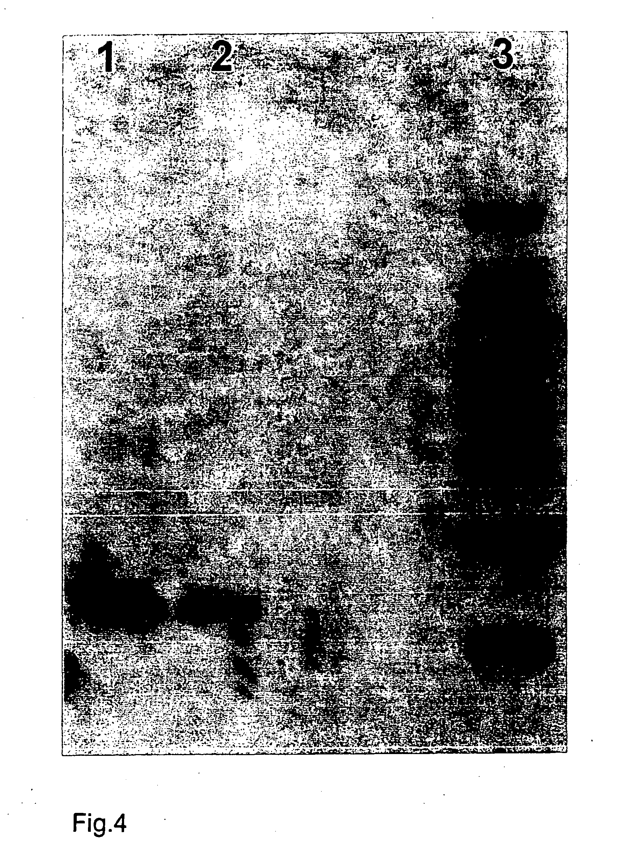

- Fig. 4: shows a Western blot of the protein expressed by the cell-free extract
1: extract with OR5 cDNA C-terminal VSV tag,
2: extract with OR5 cDNA N-terminal VSV tag, and
3: marker, SeePlus protein standard.
The Western blot shows successful transcription and translation of each of the GPCR constructs.
- Fig. 5: shows the results of radioactive detection.

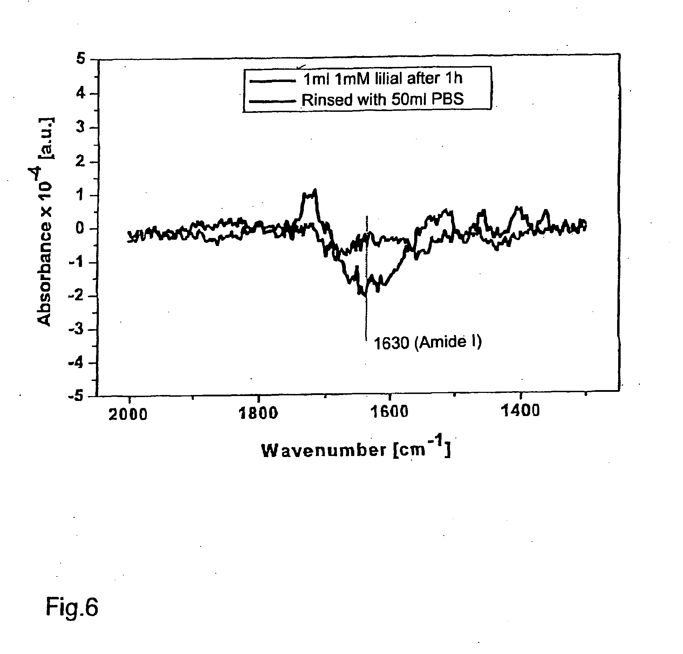

- Fig. 6: shows IR spectroscopy data of tBLM integrated OR5 protein.
- Fig. 7: shows the image of labled membrane proteins inserted into an artificial planar membrane surface.
- Fig. 8: shows results for bovine opsin on P19 tethered BLM
- Fig. 9: shows a schematic design and coding regions for the expression plasmid TNT-p3/4 including the cDNA sequence coding for bovine opsin.
- Fig. 10: shows a SPFS spectra for an incubation of tBLM with pTNT-ρ3/4.

### Examples

### Example 1

### Preparation of a peptide-tethered lipid membrane

### Assembly of peptide molecules on a planar gold surface

Peptides are used as hydrophilic spacer molecules for tethering lipid membranes. The 19-mer peptide CSRARKQAASIKVAVSADR (P19) derived from the α-laminin subunit (Sigma-Aldrich, Deisenhofen, Germany) was used. Different peptide concentrations were tested and a final concentration of 25 µg/ml in millipore water was shown to be the optimal peptide concentration in the assembling experiment, showing the association of P19 to the gold surface with respect to time/material consumption and reproducibility.

The self-assembly of a monomolecular peptide layer was enabled by the strong gold-sulfur interaction of the N-terminal cysteine moiety. The assembly of a peptide layer was completed after a 30-min incubation of the gold surface in the peptide solution. The excess unbound peptide was rinsed off and the resulting optical thickness was monitored by SPS.

The laminin peptide was covalently linked via its carboxy terminus to the primary amino moieties of phospholipid molecules (dimyristoylphosphatidylethanolamine, DMPE; 0.1 mg/ml (Sigma); solubilized in 0.001% (w/v) Triton X-100 (Roth, Karlsruhe, Germany)) by an active ester-coupling procedure. N-ethyl-N'-(dimethylaminopropyl) carbodiimide (EDC; Fluka, Deisenhofen, Germany) (11.5 mg/ml) and N-hydroxysuccinimide (NHS; Fluka) (75 mg/ml) were each dissolved in 10 ml millipore water and stored in 200-µl aliquots at -20 °C until use. EDC and NHS were mixed in equal amounts in a volume of 400 µl (50 mM NHS, 200 mM EDC) and applied onto the peptide-covered gold surface. The resulting thiopeptide-lipid layer forms a rigid assembly (approximately 1.5 nm in thickness).

To the peptide-lipid layer phosphatidylcholine vesicles were added, thereby forming a two-layer lipid membrane.

### Example 2

In vitro expression of a G-protein coupled receptor

### Components used for the experiment:

The coupled transcription and translation system (TNT®, Promega, Inc.) based on rabbit reticulocytes was employed. cDNA coding for GPCR (odorant receptor OR5 from rat) with amino-terminally or carboxy-terminally added cDNA coding for vesicular stomatitis virus glycoprotein VSV, respectively, was prepared.

Cell-free extract was applied on top of the tethered membrane produced as described in Example 1 and the cDNA construct of GPCR was added. After incubation, the surface was rinsed and primary mouse antibody, recognizing the VSV epitope, was added. The surface was rinsed again, followed by incubation with a fluorescence labeled second antibody against mouse. Increase in resulting fluorescence signal indicates the presence of VSV tag in the vicinity of the surface.

To prove the insertion of the GPCR protein in the tethered membrane, fluorescence signals deriving from various experiments were compared. Reproducible and significantly different signal strengths for amino-terminal, compared to carboxy-terminal, VSV tag were obtained. The GPCR cDNA with VSV tag at the c terminus results in five times lower signal, which equals the fluorescence signal from the TnT vector without insert as reference.

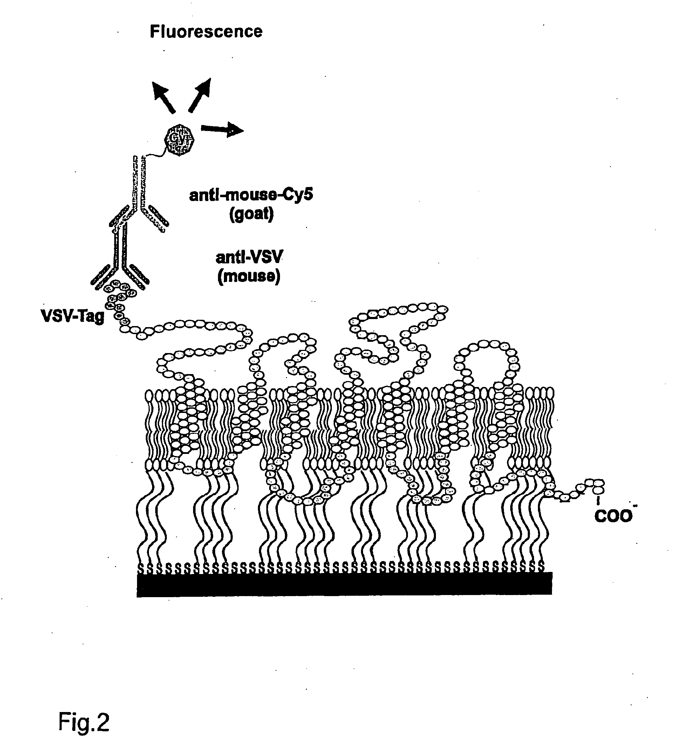

This shows the incorporation of GPCR molecules into the planar membrane system, as depicted in Fig. 2.

Proving activity of the cell-free extract and successful trancription and translation of each GPCR construct, the cell free extract was collected after incubation on the planar lipid membrane and was subjected to denaturation and prepared for western blotting.

### Example 3

Comparison between synthetic and natural membranes

When employing canine microsomes in comparison with soybean phosphatidylcholine liposomes, no significant difference in the fluorescence signals could be observed. This shows that successful protein integration takes place into the planar lipid membrane, consisting of PC only.

### Example 4

Proof of insertion using radioactive detection

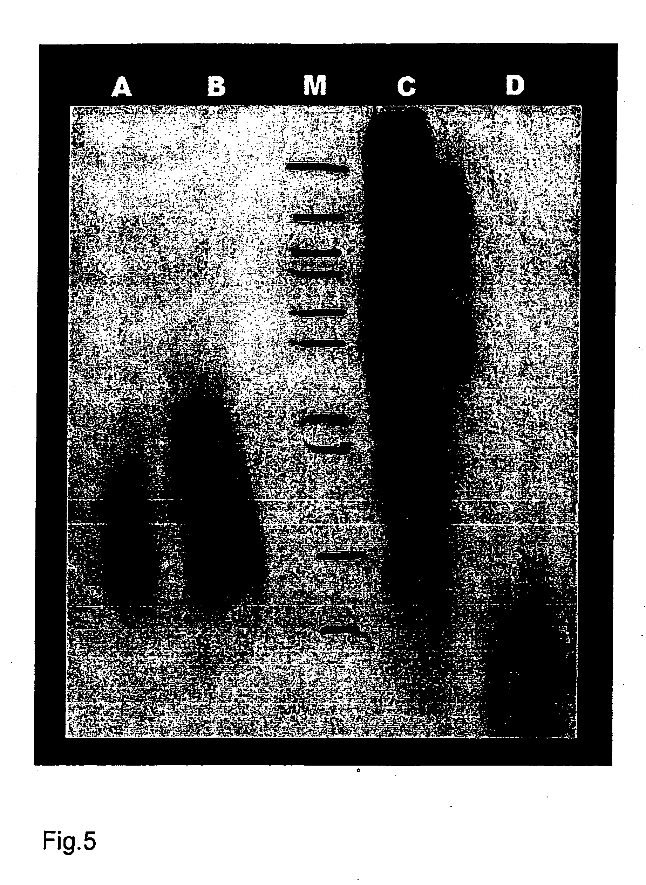

Further investigation of incorporated protein molecules has been done by radioactive labelling. Therefore we synthesized OR5 in the presence of ³⁵S-methionine. We suggested the following experiments: Due to membrane incorporation, the OR5 protein is protected against proteinase K digestion except the exposed loops from the 7TM protein. We calculated the resulting fragments in a size distribution ranging from 5,9-6,8kDa.
The results are shown in Fig. 5.

Lane A: the in vitro experiment was developed directly on the membrane surface. The proteinase K was afterwards applied. Then proteinase K and digested peptide fragments were rinsed off. The resulting membrane surface (OR5 loops) was applied onto a Acrylamide SDS gel and exposed to a film - the radioactive labeling of the protein (S was homogeneously present in the OR5 sequence) resulted in the signal in the expected range of 6kDa. Lane B: the in vitro experiment was carried out in an eppendorf tube and subsequent applied onto the membrane - expression of OR5 in the membrane resulted in the same signal, suggesting the expression in membrane fragments present in the in vitro extract and unspecifically adsorbed onto the membrane surface.
Lane M: Marker 188, 98, 62, 49, 38, 28, 17, 14, 6, 3kDa
Lane C: positive control for assay activity: in vitro extract applied onto the gel without modifications
Lane D: positive control for proteinase K activity: in vitro extract with Proteinase K incubation

### Example 5

Activity of incorporated odorant receptor

The activity of incorporated odorant receptors was measured using IR spectroscopy. The signal change of the IR amide I peak of a OR5/tBLM system upon addition of 1 mM lilial indicates recognition of the odor by the receptor. The lilial/OR5 interaction can be reversed by rinsing with PBS. Thus, the IR data show the biological activity of tBLM integrated OR5 protein.

### Example 6

Membrane protein detection by image correlation microscopy

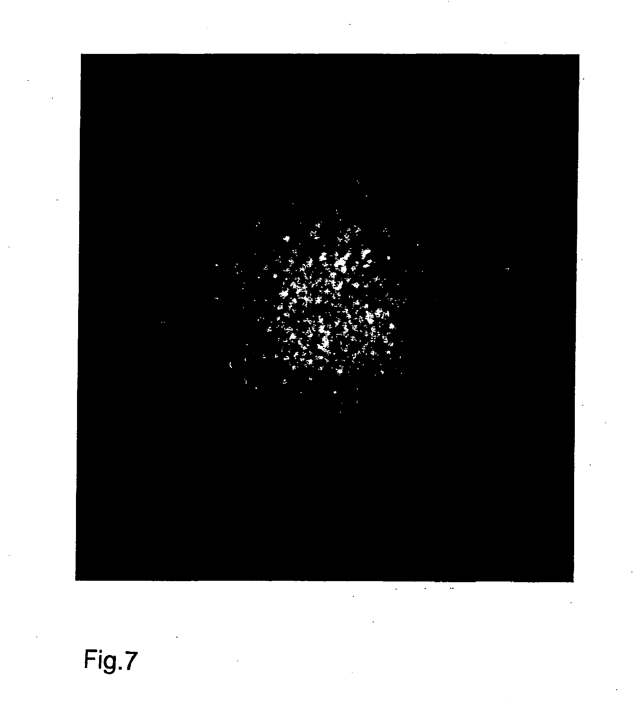

The novel method according to the invention was used for *in vitro* synthesis of complex mammalian membrane proteins into artificial planar lipid membrane structures. The cellular extract of rabbit reticulocytes contains the protein synthesis machinery for *de novo* synthesis of an olfactory receptor species starting from the mere DNA from the receptor. The density of the inserted receptor proteins by image correlation microscopy was investigated, using evanescent excitation at the surface. For instance, Figure 7 shows the fluorescently labeled antibodies tagging affinity labels of the individual membrane proteins. The excitation area has a diameter of about 20 µm. High-resolution image correlation microscopy, i.e. the spatiotemporal auto-correlation of the membrane proteins, yields information about the spatial protein distribution (incorporation density) as well as the protein mobility (diffusion in the membrane).

### Example 7

*In vitro* expression of bovine opsin on P19 tethered BLM

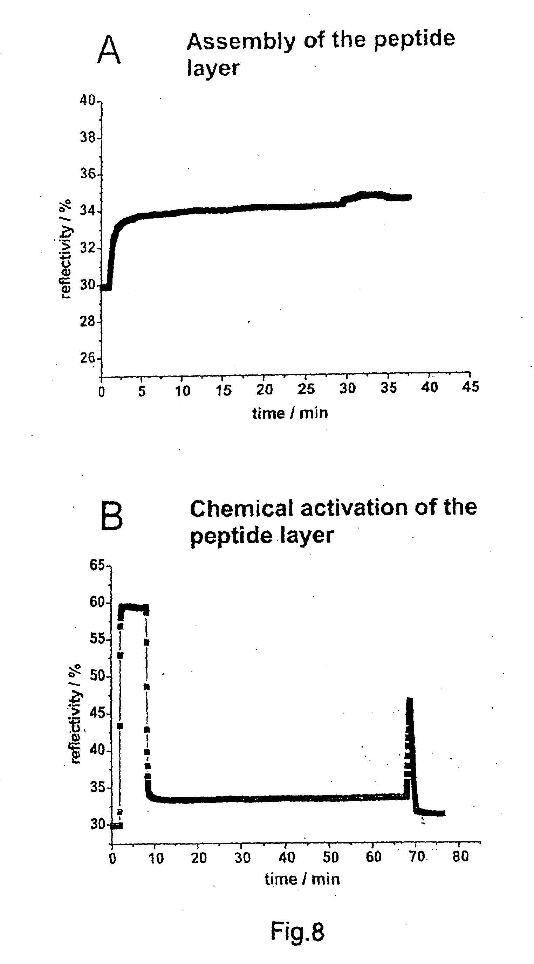

Surface architecture was assembled in the same way as described for OR5 in vitro expression / membrane insertion (Fig. 8).

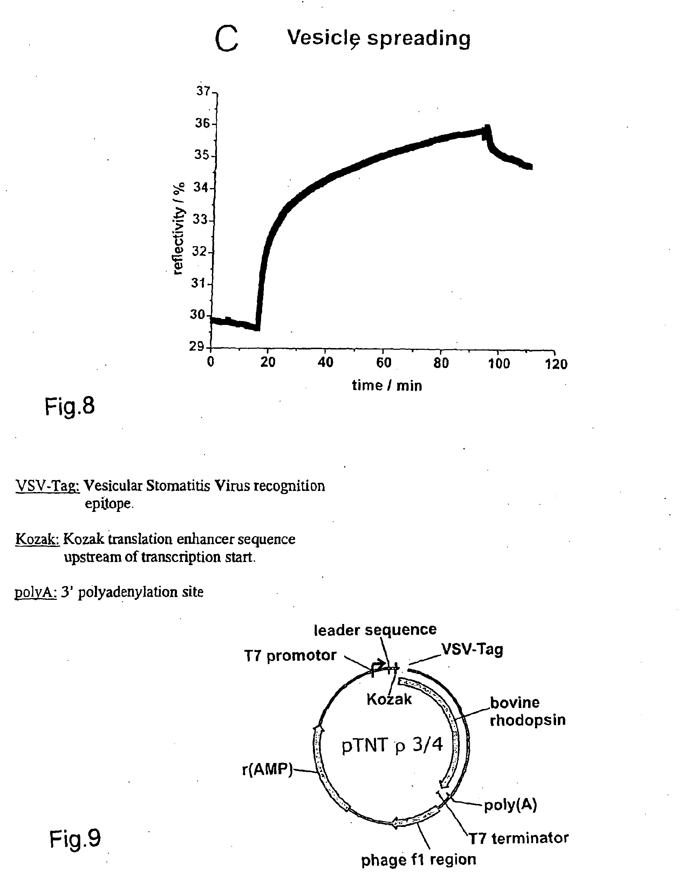

*In vitro* expression of bovine rhodopsin was performed with a T7-TNT® *in vitro* reaction mixture (Promega). The cDNA sequence coding for the bovine opsin was added to the reaction in form of the expression plasmid pTNT®-ρ3/4. Schematic design and coding regions are shown in Fig. 9.

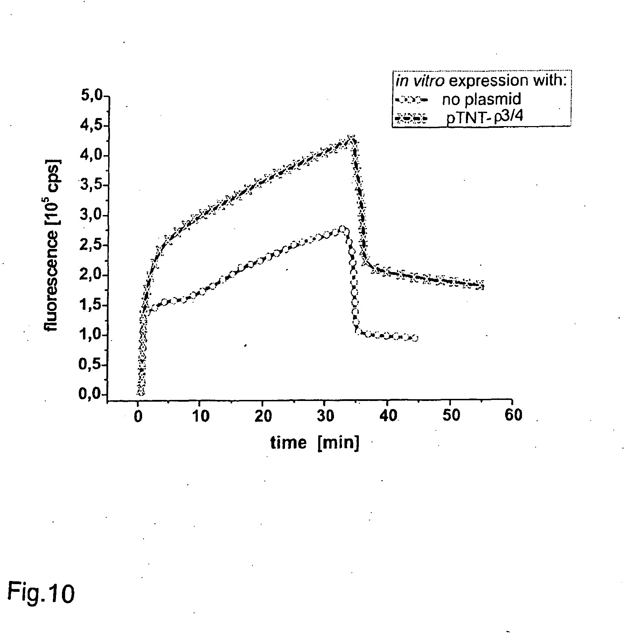

In vitro reaction mixture was incubated for 90 min at 30°C directly on top of the P19 tethered BLM attached to the SPR sensor surface. Subsequently the surface architecture was exposed to a PBS solution containing and antibody sandwich system consisting of a 1:3 mixture of monoclonal mouse anti-VSV and goat-anti mouse IgG-Cy5 antibodies. The corresponding SPFS spectra for an incubation of the tBLM with a pTNT-p3/4 containing *in vitro* expression reaction mixture and the one for an incubation with a reaction mixture containing no plasmid, respectively, is shown in Fig. 10.

## Claims

1. A process for the preparation of membrane proteins incorporated in a membrane, comprising the steps:
(i) providing a membrane, wherein the membrane is a surface bound membrane,
(ii) applying a cell-free expression system and a nucleic acid coding for the membrane proteins to the membrane, and
(iii) expression of the membrane proteins, which are integrated into the membrane.

2. The process according to claim 1, wherein the membrane is a synthetic membrane.

3. The process according to any of the preceding claims, wherein the membrane is a tethered membrane.

4. The process according to any of the preceding claims, wherein the membrane is bound or tethered to a dielectric or to a non-dielectric surface.

5. The process according to any of the preceding claims, wherein the membrane is bound or tethered to a surface consisting of a metal or metal oxide.

6. The process according to any of the preceding claims, wherein the membrane comprises natural membrane components, synthetically produced lipids or amphiphilic polymers.

7. The process according to any of the preceding claims, wherein the membrane is a two-layer lipid membrane.

8. The process according to any of the preceding claims, wherein the membrane protein is a TM protein, a membrane associated protein or a membrane spanning protein.

9. The process according to any of the preceding claims, wherein the membrane proteins are coupled with a tag, preferably with a tag selected from epitopes such as VSV, His tag, Strep tag, Flag tag, intein tag or GST tag or from a partner of a high affinity binding pair such as biotin or avidin or from a label such as a fluorescent label, an enzyme label, NMR label or isotope label.

10. The process according to any of the preceding claims, wherein the membrane proteins are tag-free.

11. The process according to any of the preceding claims, wherein the cell-free expression system is a eukaryotic cell-free expression system such as the TNT® system based on rabbit reticulocytes, a prokaryotic cell-free expression system or an archaic cell-free expression system.

## Patentansprüche

1. Verfahren zur Herstellung von Membranproteinen, die in eine Membran eingelagert sind, umfassend die Schritte:
(i) Bereitstellung einer Membran, wobei die Membran eine oberflächengebundene Membran ist,
(ii) Auftragen eines zellfreien Expressionssystems und einer für Membranproteine kodierenden Nukleinsäure auf die Membran,
(iii) Expression der Membranproteine, die in die Membran eingelagert sind.

2. Verfahren nach Anspruch 1, **dadurch gekennzeichnet, dass** die Membran eine synthetische Membran ist.

3. Verfahren nach einem der vorhergehenden Ansprüche, **dadurch gekennzeichnet, dass** die Membran eine gekoppelte Membran ist.

4. Verfahren nach einem der vorhergehenden Ansprüche, **dadurch gekennzeichnet, dass** die Membran an eine dielektrische oder nichtdielektrische Oberfläche gebunden oder gekoppelt ist.

5. Verfahren nach einem der vorhergehenden Ansprüche, **dadurch gekennzeichnet, dass** die Membran an eine Oberfläche, die aus Metall oder Metalloxid besteht gebunden oder gekoppelt ist.

6. Verfahren nach einem der vorhergehenden Ansprüche, **dadurch gekennzeichnet, dass** die Membran aus natürlichen Membrankomponenten, synthetisch hergestellten Lipiden oder amphiphilen Polymeren besteht.

7. Verfahren nach einem der vorhergehenden Ansprüche, **dadurch gekennzeichnet, dass** die Membran eine zweischichtige Lipidmembran ist.

8. Verfahren nach einem der vorhergehenden Ansprüche, **dadurch gekennzeichnet, dass** das Membranprotein ein TM Protein, ein membranassoziiertes Protein oder ein die Membran überspannendes Protein ist.

9. Verfahren nach einem der vorhergehenden Ansprüche, **dadurch gekennzeichnet, dass** die Membranproteine mit einem Tag versehen sind, vorzugsweise mit einem Tag ausgewählt aus Epitopen wie VSV, His-Tag, Strep-Tag, Flag-Tag, Intein-Tag oder GST-Tag oder ausgewählt aus einem Partner eines hochaffinen Bindungspaares wie zum Beispiel Biotin oder Avidin oder aus einer Markierung wie z.B. einer Fluoreszenzmarkierung, einer Enzymmarkierung, NMR Markierung oder einer Isotopenmarkierung.

10. Verfahren nach einem der vorhergehenden Ansprüche, **dadurch gekennzeichnet, dass** die Membranproteine keinen Tag tragen.

11. Verfahren nach einem der vorhergehenden Ansprüche, **dadurch gekennzeichnet, dass** das zellfreie Expressionssystem ein eukaryontisches zellfreies Expressionssystem wie z.B. das TNT^{®} System, basierend auf Kaninchen Retikulozyten, ein prokaryontisches zellfreies Expressionssystem oder ein archaisches zellfreies Expressionssystem ist.

## Revendications

1. Procédé de préparation de protéines de membrane incorporées dans une membrane, comprenant les étapes consistant à :
(i) fournir une membrane, dans laquelle la membrane est une membrane liée en surface,
(ii) appliquer un système d'expression acellulaire et un acide nucléique codant pour les protéines de membrane à la membrane, et
(iii) exprimer les protéines de membrane, qui sont intégrées dans la membrane.

2. Procédé selon la revendication 1, dans lequel la membrane est une membrane synthétique.

3. Procédé selon l'une quelconque des revendications précédentes, dans lequel la membrane est une membrane attachée.

4. Procédé selon l'une quelconque des revendications précédentes, dans lequel la membrane est liée ou attachée à une surface diélectrique ou non diélectrique.

5. Procédé selon l'une quelconque des revendications précédentes, dans lequel la membrane est liée ou attachée à une surface consistant en un métal ou oxyde de métal.

6. Procédé selon l'une quelconque des revendications précédentes, dans lequel la membrane comprend des composants de membrane naturels, des lipides produits synthétiquement ou des polymères amphiphiles.

7. Procédé selon l'une quelconque des revendications précédentes, dans lequel la membrane est une membrane lipidique à deux couches.

8. Procédé selon l'une quelconque des revendications précédentes, dans lequel la protéine de membrane est une protéine TM, une protéine associée à une membrane ou une protéine de recouvrement de membrane.

9. Procédé selon l'une quelconque des revendications précédentes, dans lequel les protéines de membrane sont couplées à un marqueur, de préférence un marqueur choisi parmi les épitopes tels que VSV, marqueur His, marqueur Strep, marqueur Flag, marqueur intéine ou marqueur GST, ou parmi un partenaire d'une paire de liaison de haute affinité telle que biotine ou avidine, ou parmi une étiquette telle qu'une étiquette fluorescente, une étiquette d'enzyme, une étiquette RMN ou une étiquette d'isotope.

10. Procédé selon l'une quelconque des revendications précédentes, dans lequel les protéines de membrane sont sans marqueur.

11. Procédé selon l'une quelconque des revendications précédentes, dans lequel le système d'expression acellulaire est un système d'expression acellulaire eucaryote tel que le système TNT® basé sur des réticulocytes de lapin, un système d'expression acellulaire procaryote ou un système d'expression acellulaire archaïque.
